# EUROPEAN PATENT APPLICATION

(11) **EP 4 252 763 A1**
(43) Date of publication of application: **04.10.2023**
(21) Application number: 23164942.7
(22) Date of filing: 29.03.2023
(51) Int. Cl.: A61K 35/644, A61K 47/46, C12N 5/0775

(54) **EXOSOME SECRETION PROMOTING AGENT**

(30) Priority: 30.03.2022 JP 2022056825
(71) Applicant: Yamada Bee Company, Inc., Tomata-gun, Okayama 708-0393 (JP)
(72) Inventor: OKUMURA, Nobuaki, Tomata-gun, Okayama, 708-0393 (JP); ISHIKAWA, Tomomi, Tomata-gun, Okayama, 708-0393 (JP); ITOH, Tomohiro, Tsu-shi, Mie, 514-8507 (JP)
(74) Representative: Hoffmann Eitle

(57) **Abstract**

The present invention provides an agent comprising royal jelly for use in promotion of exosome secretion by mesenchymal stem cells.

## Description

### TECHNICAL FIELD

The present invention relates to an exosome secretion promoting agent, a method for producing an exosome, and, a composition comprising an exosome.

### BACKGROUND

Exosomes are particulate matter, which have a lipid bilayer membrane structure with a diameter ranging from about 50 nm to 200 nm, and are secreted from cells. The surface of the exosomes contains cell membrane-derived lipids and membrane proteins, and the interior contains intracellular substances such as nucleic acids (microRNA (miRNA), messenger RNA (mRNA), DNA, etc.) and proteins (enzymes etc.). Through giving and receiving of exosomes, various substances such as nucleic acids and proteins can move between cells. Exosomes play an important role as a medium for cell-to-cell interaction, and have been revealed to be involved in wide-ranging biological phenomena such as cancer development/progression, immune regulation, and tissue regeneration.

In recent years, it has been revealed that exosomes secreted by mesenchymal stem cells exhibit therapeutic effects on various diseases. Further, it has been reported that exosomes derived from adipose-derived stem cells among mesenchymal stem cells have cosmetic effects such as anti-skin aging, improvement of dermatitis, and wound healing (Xiong M et al., "Exosomes From Adipose-Derived Stem Cells: The Emerging Roles and Applications in Tissue Regeneration of Plastic and Cosmetic Surgery", Front. Cell Dev. Biol., 2020; 8: 574223., Wei Zhang et al., "Cell-free therapy based on adipose tissue stem cell-derived exosomes promotes wound healing via the PI3K/Akt signaling pathway", Exp Cell Res., 201815; 370(2): 333-342. and Li Hu et al., "Exosomes derived from human adipose mensenchymal stem cells accelerates cutaneous wound healing via optimizing the characteristics of fibroblasts", Sci Rep 2016 12; 6: 32993; doi: 10.1038/srep32993.).

An exosome derived from mesenchymal stem cells can be efficiently prepared by treating mesenchymal stem cells with an agent as described in Japanese Unexamined Patent Publication No. 2020-522996, for example.

### SUMMARY

### Problems to be Solved by the Invention

An object of the present invention is to provide an exosome secretion promoting agent for efficiently producing an exosome derived from mesenchymal stem cells, and a method for producing an exosome derived from mesenchymal stem cells, particularly an exosome derived from mesenchymal stem cells having the effects of activating fibroblasts, with the use of the exosome secretion promoting agent.

### Means for Solving the Problems

As a result of intensive studies to achieve the above object, the present inventors have found that royal jelly can promote exosome secretion by mesenchymal stem cells, and, the thus obtained exosome has the high effects of activating fibroblasts, and thus have completed the present invention.

Specifically, the present invention relates to the following inventions.
[1] An exosome secretion promoting agent for promoting exosome secretion by mesenchymal stem cells, comprising royal jelly.
[2] The exosome secretion promoting agent according to [1], wherein the mesenchymal stem cells are adipose-derived mesenchymal stem cells.
[3] The exosome secretion promoting agent according to [1] or [2], improving the content of miRNA-205-5p in mesenchymal stem cells and/or an exosome secreted by mesenchymal stem cells.
[4] A cosmetic raw material, a cosmetic product, a food composition or a pharmaceutical composition, comprising the exosome secretion promoting agent according to any one of [1] to [3] as an active ingredient.
[5] A method for producing an exosome derived from mesenchymal stem cells, comprising a step of culturing mesenchymal stem cells in a medium containing royal jelly.
[6] The production method according to [5], wherein the mesenchymal stem cells are adipose-derived mesenchymal stem cells.
[7] The production method according to [5] or [6], further comprising a step of collecting a secreted exosome.
[8] A composition, comprising an exosome produced by the production method according to any one of [5] to [7].
[9] The composition according to [8], which is a skin care composition.
[10] The composition according to [8] or [9], having fibroblast activating action.
[11] The composition according to [10], wherein the fibroblast activating action includes fibroblast growth promoting action, wound healing promoting action, migration promoting action to a wound site, and/or collagen production promoting action.
[12] The composition according to any one of [8] to [11], which is a cosmetic raw material, a cosmetic product, a food composition or a pharmaceutical composition.
[13] The composition according to [12], wherein the composition is a topical composition.
[14] An agent comprising a royal jelly for use in promotion of exosome secretion by mesenchymal stem cells.
[15] The agent for use according to [14], wherein the mesenchymal stem cells are adipose-derived stem cells.
[16] The agent for use according to [14] or [15], wherein the royal jelly improves the content of miRNA-205-5p in mesenchymal stem cells and/or an exosome secreted by mesenchymal stem cells.
[17] An agent comprising a royal jelly for use in wound healing or tissue regeneration.
[18] Use of an agent comprising royal jelly for promotion of exosome secretion by mesenchymal stem cells.
[19] A cosmetic raw material, a cosmetic product, a food composition or a pharmaceutical composition, comprising a royal jelly, for use in promotion of exosome secretion by mesenchymal stem cells.
[20] Use of a cosmetic raw material, a cosmetic product, a food composition, or a pharmaceutical composition, comprising a royal jelly, for promotion of exosome secretion by mesenchymal stem cells.

### Advantageous Effects of Invention

According to the present invention, an exosome secretion promoting agent for efficiently producing an exosome derived from mesenchymal stem cells, and a method for producing an exosome derived from mesenchymal stem cells, and particularly an exosome derived from mesenchymal stem cells having fibroblast activating action, with the use of the exosome secretion promoting agent can be provided. According to the present invention, an agent comprising a royal jelly for use in promotion of exosome secretion by mesenchymal stem cells, and a cosmetic raw material, cosmetic product, food composition or pharmaceutical composition, comprising a royal jelly, for use in promotion of exosome secretion by mesenchymal stem cells can be further provided.

### BRIEF DESCRIPTION OF THE DRAWINGS

Fig. 1 shows the particle size distributions and the particle concentrations of exosomes derived from adipose-derived stem cells (ADSC) treated with various types of royal jelly in Experimental Example 1.
Fig. 2 is a graph showing the relative growth rates of ADSC treated with various types of royal jelly in Experimental Example 1.
Fig. 3 is a graph showing the relative growth rates of fibroblasts treated with various types of ADSC exosome in Experimental Example 2.
Fig. 4 is photographs showing how fibroblasts treated with various types of ADSC exosome migrate to wound sites in Experimental Example 3.
Fig. 5 is a graph showing the relative migration activity of fibroblasts treated with various types of ADSC exosome to migrate to wound sites in Experimental Example 3.
Fig. 6 is a graph showing the ability to produce collagen of fibroblasts treated with various types of ADSC exosome in Experimental Example 4.
Fig. 7 shows Venn diagrams comparing the miRNA profiles of adipose-derived stem cells and the exosome derived from adipose-derived stem cells, which were treated with freeze-dried royal jelly and enzyme-treated royal jelly in Experimental Example 5.
Fig. 8 shows Venn diagrams comparing the miRNA profiles of adipose-derived stem cells and the exosome derived from adipose-derived stem cells for each of the treatment by freeze-dried royal jelly and enzyme-treated royal jelly in Experimental Example 5.

### DETAILED DESCRIPTION

The embodiments of the present invention will be described in detail as follows, but the present invention is not limited to the following embodiments.

### [Exosome secretion promoting agent and composition comprising the same]

The exosome secretion promoting agent for promoting exosome secretion by mesenchymal stem cells of the embodiment comprises royal jelly.

Mesenchymal stem cells (MSC) are a type of somatic stem cells derived from mesodermal tissues (mesenchyme), such as bone marrow and adipose and have, in addition to self-replication ability, differentiation ability to differentiate into mesenchymal tissues such as bones, cartilages, adipose tissues, and immunoregulatory ability. The mesenchymal stem cells in the embodiment may be mesenchymal tissue-derived mesenchymal stem cells such as adipose-derived mesenchymal stem cells, bone marrow-derived mesenchymal stem cells, or dental pulp-derived mesenchymal stem cells, or may also be human induced pluripotent stem cell (hiPS cell)-derived mesenchymal stem cells (iMSC), and are preferably adipose-derived mesenchymal stem cells. The mesenchymal stem cells may be derived from any animal, but are preferably human mesenchymal stem cells.

Adipose-derived stem cells (ADSC) that can be collected from an adipose tissue is advantageous in that ADSC can be collected in a larger amount from whole-body adipose tissues compared with that of bone marrow-derived MSC. Moreover, adipose-derived MSC produce more regeneration promoting factors that contribute to organ repair than bone marrow-derived MSC. Therefore, when adipose-derived MSC are used, an exosome(s) having good quality can be produced efficiently.

Exosomes (also referred to as membrane vesicles) contain intracellular components, and are particulate extracellular vesicles that are secreted extracellularly by cells. The particle size generally ranges from 50 to 200 nm or 50 to 150 nm. The properties of exosomes differ, depending on the cell type from which exosomes are secreted. Exosomes to be secreted by mesenchymal stem cells contain: nucleic acids such as microRNA, messenger RNA and DNA; growth factors such as HGF (Hepatocyte Growth Factor), VEGF (Vascular Endothelial Growth Factor) and TGF (Tumor Growth Factor); and enzymes such as GAPDH (glyceraldehyde-3-phosphate dehydrogenase) and enolase. The exosomes to be secreted by mesenchymal stem cells are involved in wound healing, tissue regeneration, and the like by promoting cell growth (particularly, the growth of fibroblasts), and have a cosmetic effect such as anti-aging of skin.

Royal jelly is a milky gelatinous substance made by 3- to 12-day old worker bees among honeybees through mixing of a secretion secreted from the hypopharyngeal gland and the mandibular gland. Examples of major biologically active components in royal jelly include organic acids such as 10-hydroxy-2-decenoic acid, and 10-hydroxydecanoic acid unique to royal jelly, a protein, an amino acid, a peptide, a lipid, sugars, vitamins such as vitamin Bs, a folic acid, a nicotinic acid, and a pantothenic acid, and various types of minerals.

In the present specification, the royal jelly may be, for example, raw royal jelly, or may be a royal jelly treated product prepared by subjecting raw royal jelly to treatment.

The raw royal jelly can be obtained for example as a beekeeping product in accordance with a conventional method. A place of the production of royal jelly is not limited, and may be any of Japan, China, Brazil, European countries, Oceanian countries, the U.S.A., and the like. Examples of raw royal jelly include frozen royal jelly prepared by cryopreservation of collected royal jelly.

Examples of treated royal jelly include: royal jelly concentrates or dilutions prepared by concentrating or diluting raw royal jelly; royal jelly powder prepared by drying and powdering raw royal jelly; royal jelly organic solvent extracts such as royal jelly ethanol extracts prepared by extracting royal jelly with an organic solvent such as ethanol; and enzymatically decomposed royal jelly prepared by treating royal jelly with a protease. The treated royal jelly may be royal jelly having been subjected to a plurality of treatments. The royal jelly may be an enzymatically decomposed royal jelly powder obtained by enzymatic decomposing and powderization. The treated royal jelly may be preferably enzyme-treated royal jelly, and more preferably enzyme-treated royal jelly powder.

The royal jelly concentrates can be obtained by, for example, removing moisture content from raw royal jelly. The royal jelly dilutions can be obtained by, for example, adding water to raw royal jelly.

The royal jelly powder can be obtained by, for example, powderizing raw royal jelly through a method known in this technical field such as freeze-drying and spray drying, and the like. As a drying method, any known method that is employed in general food processing, such as natural drying, e.g., ventilation drying and solar drying, forced drying that involves heating with electricity or the like for drying, and freeze-drying can be used. Preferably freeze-drying is used. Note that the time for drying is not particularly limited, and examples thereof can include about 3 days in the case of natural drying such as ventilation drying or solar drying, and about 1 to 3 days at about 50°C in the case of forced drying via heating with electricity or the like. Usually, drying is preferably performed in such a manner that the water content is 10 mass% or less, and preferably 5 mass% or less. Note that if it is difficult to lower the water content to 10 mass% or less as in the case of natural drying such as ventilation drying or solar drying, the dried product may be further subjected to treatment with a freeze-dryer for further lowering the water content. In addition, royal jelly powder may be obtained through pulverization using a pulverizer (for example, a pin mill, a hammer mill, a ball mill, and a jet mill) after freeze-drying or spray-drying.

The royal jelly organic solvent extract can be obtained by, for example, extracting raw royal jelly or royal jelly powder using, as a solvent, an organic solvent such as ethanol, methanol, propanol or acetone. The extraction time can be appropriately set according to the form of the raw royal jelly used as a starting material, the type and the amount of the solvent, the temperature and the stirring conditions in the extraction, etc. After the extraction, a solid matter may be removed by filtration, centrifugation or the like. The extracted solution may be used as it is, or the solution may be used as a concentrated solution or a powder by removing the solvent from the solution. It is preferable that the royal jelly organic solvent extract be a royal jelly ethanol extract.

The enzymatically decomposed royal jelly can be obtained by, for example, treating raw royal jelly or royal jelly powder with protease. Examples of protease is preferably selected from the group consisting of an enzyme having endopeptidase action, an enzyme having exopeptidase action, and an enzyme having both endopeptidase action and exopeptidase action. In particular, protease is preferably a peptidase simultaneously having both endopeptidase action and exopeptidase action. Enzymatic treatment using such peptidase can lower the molecular weight of a protein with one-step enzymatic treatment, and thus is advantageous in that the operation is simple, as well as the disappearance of and a significant reduction in the bioactivity of useful components contained in royal jelly can be prevented.

The origin of protease is not particularly limited, and peptidases derived from animals, plants and microorganisms (bacteria, viruses, fungi (mold, yeast, mushroom etc.), algae and the like) can be widely used.

The "exopeptidase" is categorized into "aminopeptidase" and "carboxypeptidase". Further, regarding peptidase, a term of acid, neutral, or alkaline may be attached to each "enzyme" depending on the optimum pH, and thus peptidase may also be referred to as "acid exopeptidase", "neutral aminopeptidase", or "alkaline endopeptidase", for example.

Examples of protease having at least endopeptidase activity include animal-derived (for example, trypsin, or chymotrypsin), plant-derived (for example, papain), microorganism-derived (for example, lactic acid bacteria, yeast, mold, *Bacillus subtilis,* or actinomycete) endopeptidases.

Examples of protease having at least exopeptidase activity include carboxypeptidase, aminopeptidase, microorganism-derived (for example, lactic acid bacteria, microorganisms of the genus *Aspergillus,* microorganisms of the genus *Rhizopus* etc.) exopeptidase, pancreatin also having endopeptidase activity, and pepsin.

Preferred examples of enzymes having both exopeptidase activity and endopeptidase activity can include *Streptomyces griseus-*produced peptidase (trade name: Actinase AS), *Aspergillus oryzae-*produced peptidase (trade name: Protease A, Flavourzyme, Proteax, Sumizyme LP-G), and *Aspergillus melleus-produced* peptidase (trade name: Protease P).

Further, preferred examples of enzymes having exopeptidase activity can include *Aspergillus oryzae*-produced peptidase (trade name: Umamizyme G, Promod 192P, Promod 194P, Sumizyme FLAP), *Aspergillus sojae*-produced peptidase (trade name: Sternzyme B15024), the genus *Aspergillus*-produced peptidase (trade name: Kokulase P), and *Rhizopus oryzae*-produced peptidase (trade name: peptidase R).

Further, preferred examples of enzymes having endopeptidase activity can include *Bacillus subtilis*-produced peptidase (trade name: Orientase 22BF, Nucleicin), *Bacillus licheniformis-*produced peptidase (trade name: Alcalase), *Bacillus stearothermophilus-*produced peptidase (trade name: Protease S), *Bacillus amyloliquefaciens-*produced peptidase (trade name: Neutrase), and the genus *Bacillus-*produced peptidase (trade name: Protamex).

Reaction conditions (the amount of protease to be used, temperature and pH at the time of reaction, reaction time, etc.) when raw royal jelly is treated with protease may be appropriately set, depending on the type of protease to be used, and the like.

Commercially available royal jelly may also be used. Specific examples of commercially available royal jelly include Enzyme-treated Royal Jelly King (produced by Yamada Bee Company, Inc.) and the like.

When mesenchymal stem cells are cultured in the presence of royal jelly, the amount of exosome secretion by mesenchymal stem cells is promoted. The amount of the thus promoted exosome secretion may increase by 10% or more, preferably 20% or more, 30% or more, 50% or more, 70% or more, 100% or more, 150% or more, or 200% or more, based on the number of particles of the exosome, or the protein per unit volume of the culture supernatant, compared with a case where royal jelly is absent.

In a case of culturing mesenchymal stem cells in the presence of royal jelly, components in mesenchymal stem cells can be changed, compared with a case where royal jelly is absent. In particular, the microRNA (miRNA) profile is changed, and of these, particularly the miRNA-205-5p content (content ratio) in total microRNA is improved. Similarly, in a case of culturing mesenchymal stem cells in the presence of royal j elly, components in the exosome secreted by mesenchymal stem cells can be changed, compared with a case where royal jelly is absent. Particularly the microRNA (miRNA) profile in the exosome derived from mesenchymal stem cells is changed, and of these, particularly the miRNA-205-5p content (content ratio) in total microRNA is improved. Accordingly, the exosome secretion promoting agent of the embodiment has an effect of improving the miRNA-205-5p content in mesenchymal stem cells and/or the exosome secreted by mesenchymal stem cells.

An improvement of the content (content ratio) of the miRNA-205-5p may be, compared with a case where royal jelly is absent, increased by 10% or more, preferably, increased by 20% or more, 30% or more, 50% or more, 70% or more, 100% or more, 150% or more, or 200% or more of the content of the miRNA-205-5p with respect to total miRNA in mesenchymal stem cells. Moreover, compared with a case where royal jelly is absent, the improvement of the content of the miRNA-205-5p may be increased by 10% or more, preferably 20% or more, 30% or more, 50% or more, 70% or more, 100% or more, 150% or more, or 200% or more of the content of the miRNA-205-5p with respect to total miRNA in the exosome derived from mesenchymal stem cells.

The exosome secretion promoting agent of the embodiment can be used for producing *ex vivo* an exosome derived from mesenchymal stem cells (for example, used for a method for producing an exosome derived from mesenchymal stem cells described later), and, can also be administered to a human for use in *in vivo* promotion of the secretion of an exosome derived from mesenchymal stem cells. The exosome secretion promoting agent of the embodiment may be administered orally or parenterally. Examples of oral administration include enteral administration, and examples of parenteral administration include topical administration and particularly include transdermal administration.

The dose of the exosome secretion promoting agent of the embodiment in the case of oral administration can differ, depending on the form and application method/amount to be applied of the composition, and may range from 10 to 30,000 mg of royal jelly, preferably 100 to 20,000 mg, 150 mg to 15,000 mg, 600 mg to 12,000 mg, 1,200 mg to 10,000 mg, or 2,400 to 8,000 mg of royal jelly in terms of dry solid content per day for an adult weighing 60 kg. This content can be appropriately increased or decreased depending on factors including the health status of a person who takes the agent, the method for administration, combinations with other agents, and the like. The exosome secretion promoting agent according to the embodiment may be administered once a day, or separately in divided doses such as twice a day, three times a day, and the like, as long as the effective dose per day is within the above ranges. The effect of the exosome secretion promoting agent of the embodiment can be obtained immediately after administration, but consecutive administration of the same for 1 to 4 weeks, or 1 month or more, 6 months or more, or 1 year or more is preferable since the effect can be sustained.

The dose of the exosome secretion promoting agent of the embodiment in the case of parenteral administration can differ depending on a site for application and the range of application, and, for example, the amount of the agent to be applied to skin may range from 0.01 to 50 mg of royal jelly, preferably 0.02 to 40 mg, 0.02 to 35 mg, or, 0.025 to 30 mg of royal jelly in terms of dry solid content.

One embodiment of the present invention provides a composition comprising the exosome secretion promoting agent of the above embodiment as an active ingredient, wherein the composition may be an oral composition or a topical composition, and particularly provides a cosmetic raw material, a cosmetic product, a food composition or a pharmaceutical composition comprising the exosome secretion promoting agent of the above embodiment as an active ingredient. The exosome secretion promoting agent or the cosmetic raw material, cosmetic product, food composition or pharmaceutical composition of the embodiment is applied to a human to act on mesenchymal stem cells in the human body, thereby promoting the amount of an exosome to be secreted, increasing the amount of the exosome derived from mesenchymal stem cells *in vivo,* and obtaining various cosmetic effects and immune control effects. Moreover, skin care effects and effects of activating fibroblasts described later are also obtained.

The royal jelly content in the cosmetic raw material, cosmetic product, food composition or pharmaceutical composition comprising the exosome secretion promoting agent of the embodiment is not particularly limited, and may be an effective dose, with which the doses of the exosome secretion promoting agent for oral administration or parenteral administration can be achieved.

The exosome secretion promoting agent in mesenchymal stem cells, or the cosmetic raw material, cosmetic product, food composition or pharmaceutical composition comprising the exosome secretion promoting agent according to the embodiment may further contain other components in addition to royal jelly as an active ingredient. Examples of other components can include pharmacologically acceptable components (for example, an excipient, a binding material, a lubricant, a disintegrator, an emulsifier, a surfactant, a base, a solubilizer, and a suspending agent), components acceptable as foods (for example, minerals, vitamins, flavonoids, quinones, polyphenols, an amino acid, a nucleic acid, an essential fatty acid, a refreshing agent, a binding agent, a sweetener, a disintegrator, a lubricant, a colorant, perfume, a stabilizing agent, an antiseptic, a sustained release adjusting agent, a surfactant, a solubilizer, and a wetting agent), and components acceptable as cosmetics (for example, a skin-lightening agent, a moisturizer, an antioxidant, an oily component, a UV absorber, a surfactant, a thickener, alcohols, a powder component, a color material, an aqueous component, water, various skin nutrients).

The exosome secretion promoting agent in mesenchymal stem cells, or the food composition or pharmaceutical composition comprising the exosome secretion promoting agent according to the embodiment may be in any form such as a solid, a liquid, and a paste, and may also be in any dosage form such as tablets (including uncoated tablets, sugar coated tablets, effervescent tablets, film coated tablets, chewable tablets, and lozenges), capsules, pills, powdered drugs (powders), fine granules, granules, liquid agents, suspensions, emulsions, syrups, pastes, injections (including a case in which the agent or the composition is mixed with distilled water or an infusion such as an amino acid infusion or an electrolyte infusion to prepare a liquid agent, when used), or a topical dosage form such as a liniment or a patch. These various formulations can be prepared, for example, by mixing active ingredients with other components as necessary and thus forming the above dosage forms.

The food composition is preferably a food having an enhanced tertiary food function (body condition-controlling function). Examples of such a food having an enhanced tertiary food function can include health foods, foods with function claims, foods with functional claims, nutritional supplements, supplements and foods for specified health uses.

The forms of the food composition are not particularly limited, and may be, for example, beverages (refreshing drinks such as coffee, juices, and tea beverages, milk beverages, lactic acid bacteria beverages, yogurt beverages, carbonated beverages, etc.); spreads (custard cream, etc.); pastes (fruit pastes etc.); Western confectioneries (chocolates, doughnuts, pies, cream puffs, chewing gums, jellies, candies, cookies, cakes, puddings etc.); Japanese confectioneries ("daifuku" (rice cakes stuffed with bean jam), rice cakes, sweet buns, Castella (sponge cakes), "anmitsu" (classic Japanese desert), "yokan" (a sweet jelly of beans), etc.); frozen desserts (ice creams, ice candies, sherbets, etc.); cooked foods (curries, "gyudon" (a bowl of rice topped with beef), a porridge of rice and vegetables, miso soups, soups, meat sauces, pastas, salted vegetables, jams, etc.); or flavoring agents (dressings, rice seasonings, umami flavoring agents, soup stocks, etc.).

The cosmetic raw material may be in any form such as a solid, a liquid, or a paste. The cosmetic product may be a medicinal cosmetic product (that is, a quasi-drug). Examples of the cosmetic product include all cosmetic products applicable to sites such as skin, mucous membranes, hair, scalp hair, scalp, nails, teeth, facial skin, lips and the like of animals (particularly, humans).

The dosage form of the cosmetic product may be a solubilization-based, an emulsification-based, a powder-based, an oily-liquid-based, a gel-based, an ointment-based, an aerosol-based, a water-oil two-layer-based, or a water-oil-powder three-layer-based form, for example. The cosmetic products may be, for example, skin care items such as a face wash, a face lotion, a milky lotion, a cream, a gel, an essence, a serum, a pack, a mask, a mist, and a UV protective cosmetic product, make up cosmetic products such as a foundation, a lip stick, rouge, an eye shadow, an eye liner, and a mascara, face washes, massage agents, cleansing agents, after shave lotions, pre-shave lotions, shaving creams, body soap, soap, shampoos, rinses, hair treatments, hair preparations, hair tonics, hair mists, hair foams, hair liquids, hair gels, hair sprays, hair growth agents, antiperspirants, bath salts or bath bombs, mouth rinses, oral cosmetic products, dentifrices, hand creams, hand soaps, or the like.

### [Method for producing exosome derived from mesenchymal stem cells]

The method for producing an exosome derived from mesenchymal stem cells of the embodiment comprises a step of culturing mesenchymal stem cells in a medium containing royal jelly.

A medium for culturing mesenchymal stem cells is not particularly limited, as long as the medium is appropriate for culturing mesenchymal stem cells, and examples thereof include an HDF medium (Lonza K.K.). Further, if necessary, a component such as a basic fibroblast growth factor (bFGF) may also be added. Mesenchymal stem cells may be cultured under usual culture conditions of, for example, 30 to 37°C (particularly, 37°C) and 3 to 5%CO₂ (particularly, 5%CO₂).

The amount of royal jelly contained in the medium is not particularly limited, as long as it is an effective dose, with which exosome secretion can be promoted in mesenchymal stem cells, and may range from 50 to 5,000 µg/mL, 50 to 1,000 µg/mL, 50 to 500 µg/mL, 100 to 300 µg/mL, or the like in terms of dry solid content with respect to the total amount of the medium. The time for culturing may be any time as long as a desired amount of an exosome is secreted, and may be 1 to 10 days or 1 to 5 days, for example. Medium exchange may be performed as necessary.

The method for producing an exosome derived from mesenchymal stem cells of the embodiment may further comprise a step of collecting the thus secreted exosome. Since the exosome is secreted in a medium (culture supernatant) after the above step of culturing, the culture supernatant is recovered by centrifugation or filtration, so that the exosome derived from mesenchymal stem cells can be collected. Furthermore, unnecessary components in the supernatant are removed and then the exosome may be purified. Purification is performed by removing cell debris in the culture supernatant by high-speed centrifugation, centrifuging the obtained supernatant using an ultracentrifuge (for example, 100,000 rpm, 60 minutes), and then collecting the exosome as a precipitate. The collected exosome may be applied to a size-exclusion column or the like in order to adjust the particle size.

The exosome obtained by the production method according to the embodiment has a particle size ranging from 30 to 500 nm, and contains components such as nucleic acids (mRNA, microRNA) and proteins. Of these, particularly miRNA-205-5p content in total microRNA may increase by 10% or more, preferably, 20% or more, 30% or more, 50% or more, 70% or more, 100% or more, 150% or more, or preferably 200% or more compared with a case in which royal jelly is absent. The exosome derived from mesenchymal stem cells obtained by this production method has skin care action and fibroblast activating action described later.

### [Exosome derived from mesenchymal stem cells and composition comprising the same]

The exosome derived from mesenchymal stem cells of the embodiment is an exosome obtained by the above method for producing an exosome derived from mesenchymal stem cells, and may be a culture supernatant containing the exosome derived from mesenchymal stem cells or a purified exosome derived from mesenchymal stem cells. One embodiment of the present invention also provides a composition comprising the exosome derived from mesenchymal stem cells of the embodiment. The composition may be an oral composition or a topical composition.

The culture supernatant containing the exosome derived from mesenchymal stem cells of the embodiment contains, for example, 5.00 × 10⁶ particles/mL or more of the exosome derived from mesenchymal stem cells, and may be the one containing 2.00 × 10⁷ particles/mL or more, 5.00 × 10⁷ particles/mL or more, 2.00 × 10⁸ particles/mL or more of the exosome derived from mesenchymal stem cells.

The exosome derived from mesenchymal stem cells of the embodiment has fibroblast activating action. Specifically, the exosome has fibroblast growth promoting action, wound healing promoting action, migration promoting action to a wound site, and/or collagen production promoting action. Therefore, a composition comprising the exosome derived from mesenchymal stem cells can be used as a skin care composition. Examples of skin care may include suppressing wrinkles, preventing the thinning of skin, delaying skin aging, and promoting skin turnover.

The composition comprising the exosome derived from mesenchymal stem cells of the embodiment may be a cosmetic raw material, a cosmetic product, a food composition or a pharmaceutical composition, the form/dosage form and the route of administration of the composition can be the same as those of the above composition comprising the exosome secretion promoting agent except that the active ingredient is the exosome derived from mesenchymal stem cells, and the composition may comprise similar components other than the active ingredient. Moreover, the composition comprising the exosome derived from mesenchymal stem cells of the embodiment may be a topical composition, and the topical composition can be a cosmetic composition or a pharmaceutical composition. The topical composition may be a percutaneous pharmaceutical composition (external preparation for skin), such as a liniment (for example, an ointment, a cream, and a liquid for external use), and a patch. When the topical composition is a cosmetic product, it may be the above face lotion, milky lotion, cream, gel, essence, serum or the like.

The composition comprising the exosome derived from mesenchymal stem cells of the embodiment may comprise the exosome derived from mesenchymal stem cells as an active ingredient. The effective dose of the above composition can differ depending on the form and application method/amount applied of the composition, but may range from 10 to 30,000 mg, preferably 100 to 20,000 mg, 150 mg to 15,000 mg, 600 mg to 12000 mg, 1,200 mg to 10,000 mg, or, 2,400 to 8,000 mg per day for an adult weighing 60 kg. This capacity can be appropriately set within the above ranges depending on factors including the health status of a person who takes the composition, the administration method, a combination with other agents, and the like. The composition comprising the exosome derived from mesenchymal stem cells of the embodiment may be administered once a day, or administered separately in divided doses, such as twice a day, and three times a day, as long as the effective dose per day is within the above ranges. The composition comprising the exosome derived from mesenchymal stem cells of the embodiment can exhibit the effect immediately after administration, but the consecutive administration of the composition, such as administration for 1 to 4 weeks, or 1 month or more, 6 months or more, and 1 year or more, is preferable since the effect can be sustained longer.

The dose of the composition comprising the exosome derived from mesenchymal stem cells of the embodiment in the case of parenteral administration can differ depending on a site for application and the application range thereof, but, for example, the amount of the composition to be applied to skin may range from 0.01 to 50 mg, 0.02 mg to 40 mg, 0.02 to 35 mg, or, 0.025 to 30 mg in terms of dry solid content.

### [Use for promotion of exosome secretion]

One embodiment provides an agent comprising a royal jelly for use in promotion of exosome secretion by mesenchymal stem cells. Here, the above mesenchymal stem cells may be adipose-derived stem cells, and the above royal jelly preferably improves the miRNA-205-5p content in the above mesenchymal stem cells and/or exosome to be secreted by the mesenchymal stem cells. The embodiment also provides an agent comprising a royal jelly for use in wound healing or tissue regeneration. The embodiment further provides use of an agent comprising royal jelly for promotion of exosome secretion by mesenchymal stem cells.

One embodiment provides a cosmetic raw material, a cosmetic product, a food composition or a pharmaceutical composition comprising the royal jelly for use in promotion of exosome secretion by mesenchymal stem cells. The embodiment also provides use of a cosmetic raw material, a cosmetic product, a food composition, or a pharmaceutical composition, comprising a royal jelly, for promotion of exosome secretion by mesenchymal stem cells.

Hereinafter, the present invention will be more specifically described based on Examples. However, the present invention is not limited to the following Examples.

### EXAMPLES

### (Experimental Example 1 Evaluation of the effects of treatment with various types of royal jelly on exosome secretion)

### [Evaluation of the particle sizes and particle concentrations of exosomes]

Adipose-Derived Stem Cells (hereinafter, also abbreviated as "ADSC") purchased from Lonza K.K. were used. ADSC prepared at 1 × 10⁵ cells/mL were seeded in a T75 flask, and then pre-cultured in ADSC medium (Lonza K.K., MSCBM (Registered Trademark) basal medium) at 37°C and 5%CO₂ for 48 hours. After 48 hours, freeze-dried royal jelly (FDRJ, Yamada Bee Company, Inc., royal jelly subjected to freeze-drying and powderization) or enzyme-treated royal jelly (EzRJ, Yamada Bee Company, Inc., royal jelly subjected to enzymatic decomposing and then powderization) was added to the medium in such a manner that the concentration was 100 µg/mL, followed by 5 days of culture. After 5 days of culture, the medium was recovered, and then exchanged with the same, but newly prepared medium, followed by 5 days of culture. After culture, the medium was recovered and then combined with the medium recovered after the first 5 days of culture.

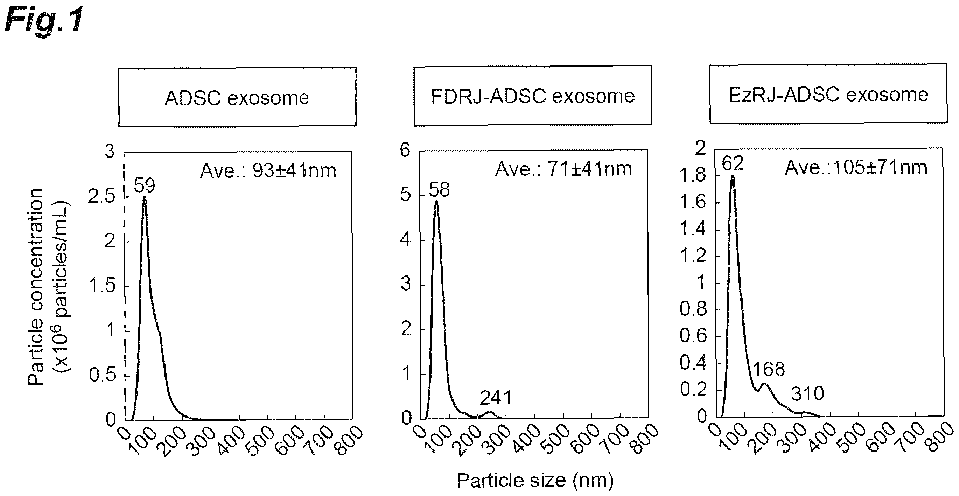

The medium was subjected to centrifugation at 15,000 rpm and 4°C for 30 minutes to remove dead cell debris and the like in the above mentioned recovered medium. After centrifugation, a supernatant liquid was recovered, further subjected to ultracentrifugation at 100,000 rpm and 4°C for 60 minutes, thereby precipitating an exosome (ADSC exosome) derived from adipose-derived stem cells. The precipitate was suspended in cold phosphate-buffered saline (PBS), and then the suspension was applied to a size exclusion column qEV70nm (Meiwafosis Co., Ltd.), thereby obtaining an ADSC exosome. The ADSC-derived exosome treated with FDRJ or EzRJ is referred to as FDRJ-ADSC exosome or EzRJ-ADSC exosome, and control ADSC-derived exosome not treated with royal jelly is referred to as untreated ADSC exosome or simply ADSC exosome. The particle size and particle concentration of each exosome type were calculated using Nanoparticle tracking analysis (NTA, Version 2.3) based on moving images recorded for observation of Brownian movement with Nanosight LM14C. Fig. 1 shows the results.

According to Fig. 1, the peak of the particle size of untreated ADSC exosome was at 59 nm, and the mean particle size was 93±41 nm. The peak of the particle size of FDRJ-ADSC exosome was at 58 nm, and the mean particle size was 71±41 nm. Further, the peak of the particle size of EzRJ-ADSC exosome was at 62 nm, and the mean particle size was 105±71 nm. All of these exosomes had almost the same particle sizes. Further, regarding the particle concentration per recovered medium (exosome concentration), the particle concentration of untreated ADSC exosome was 4.05 × 10⁶ particles/mL, that of FDRJ-ADSC exosome was 2.01 × 10⁸ particles/mL, and that of EzRJ-ADSC exosome was 2.22 × 10⁷ particles/mL (Fig. 1). Compared with the concentration of untreated ADSC exosome, the concentration of FDRJ-ADSC exosome increased about 50 times, and the concentration of EzRJ-ADSC exosome increased about 5.5 times.

### [Evaluation of ADSC growth rate]

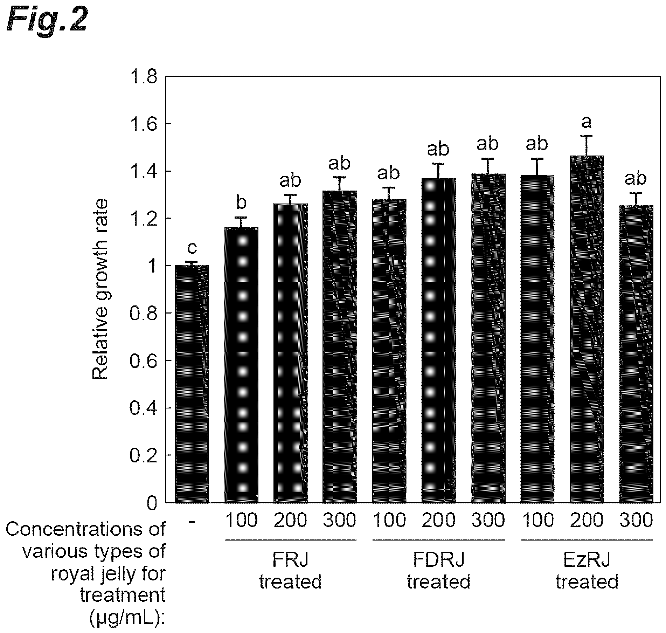

ADSC adjusted at 1 × 10⁴ cells/mL were seeded on a 96-well plate at 100 µL/well, and then pre-cultured for 24 hours in the same manner as described above. After 24 hours, the media were exchanged with ADSC media (Lonza K.K.) containing frozen royal jelly (FRJ, Yamada Bee Company, Inc., raw royal jelly), FDRJ, and EzRJ, at 100 µg/mL, 200 µg/mL, and 300 µg/mL, respectively, followed by 72 hours of culture. After 72 hours of culture, the media were exchanged with 10% WST-1 (Takara Bio Inc.)-containing ADSC media, followed by 2 hours of culture. After the culture, the absorbance of a formazan dye converted from WST-1 by succinate-tetrazolium reductase functioning in the mitochondrial respiratory chain was measured at 450 nm and 690 nm with a microplate reader, and then based on the absorbance, the relative growth rate of ADSC when the growth rate of untreated ADSC was determined to be 1 was calculated. Fig. 2 shows the results. Note that a, ab, b, and c in Fig. 2 indicate that there is a significant difference between different symbols. Further, a and ab, and, b and ab share the same symbol, indicating there is no significant difference.

Fig. 2 demonstrates that the growth of ADSC treated with various types of royal jelly significantly increased compared with that of untreated ADSC. For example, the relative growth rates of ADSC treated with various types of royal jelly at 100 µg/mL were 1.16 (FRJ), 1.28 (FDRJ), and 1.38 (EzRJ), respectively.

Based on the above two evaluation results, when the results such that the concentration of ADSC exosome increased about 50 times in the case of treatment with FDRJ, and increased about 5.5 times in the case of treatment with EzRJ, compared to the untreated case were considered together with the results of the ADSC growth rates, the results of both the treatment with FDRJ and the treatment with EzRJ cannot be explained based on only such increases in the number of cells. Hence, treatment with various types of royal jelly was considered to cause the promoted secretion of ADSC exosome from ADSC.

### (Experimental Example 2 Evaluation of the effects of various types of ADSC exosome on the growth of fibroblasts)

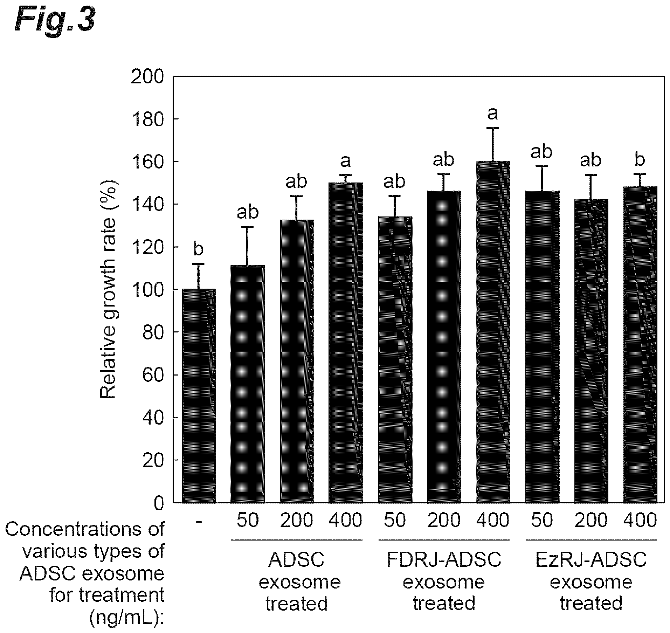

Normal Human Dermal Fibroblasts (hereinafter, also referred to as "NHDF") purchased from Lonza K.K. were used. NHDF prepared at 1 × 10⁴ cells/mL were seeded in a 96-well plate at 100 µL/well, and then cultured in NHDF media (Lonza K.K., MSCBM (Registered Trademark) basal medium) at 37°C and 5%CO₂ for 24 hours. After 24 hours, the media were exchanged with NHDF media, to which ADSC exosome, FDRJ-ADSC exosome, and EzRJ-ADSC exosome had been added in such a manner that the concentrations were 50, 200, and 400 ng protein/mL, respectively, and then NHDF were cultured for 72 hours. After 72 hours of culture, the media were exchanged with 10% WST-1 (Takara Bio Inc.)-containing NHDF media, followed by 2 hours of culture. Further after the culture, the absorbance was measured in the same manner as in [Evaluation of ADSC growth rate] above, and then based on the absorbance, the relative growth rate (%) of NDHF when the cell growth rate of untreated NHDF was determined to be 100% was calculated. Fig. 3 shows the results. Note that a, ab, and b in Fig. 3 indicate that there is a significant difference between different symbols. Further, a and ab, and, b and ab share the same symbol, indicating there is no significant difference.

Fig. 3 demonstrates that both treatment with FDRJ-ADSC exosome and treatment with EzRJ-ADSC exosome resulted in significant increases in NHDF growth rate at the treatment concentration of 400 ng protein/mL. Further, the treatment at a concentration of 50 or 200 ng protein/mL did not affect the NDHF growth rates. No significant difference was observed in NHDF growth rate between treatment with ADSC exosome and treatment with ADSC exosome treated with various types of royal jelly.

### (Experimental Example 3 Evaluation of the effects of various types of ADSC exosome on the migration activity of fibroblasts to wound sites)

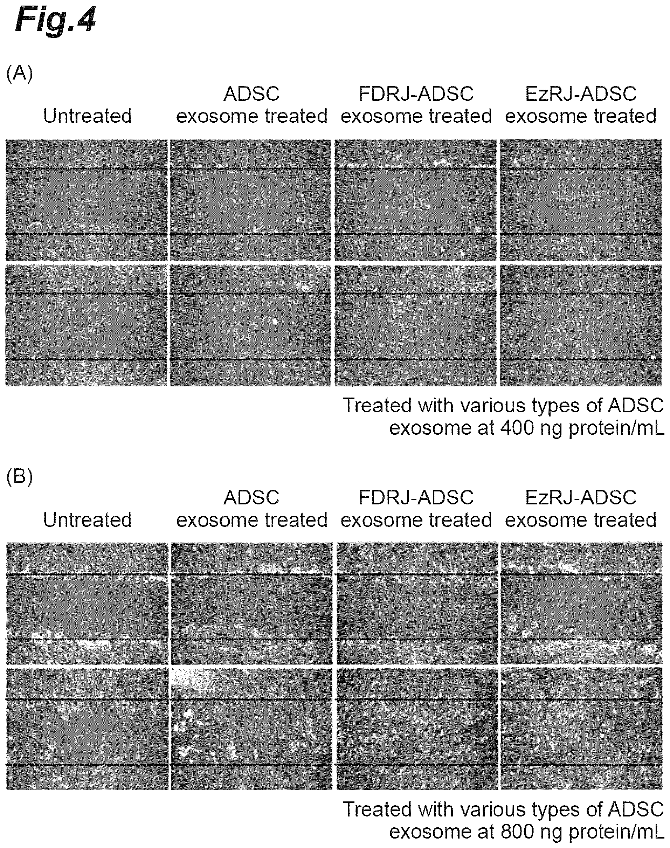

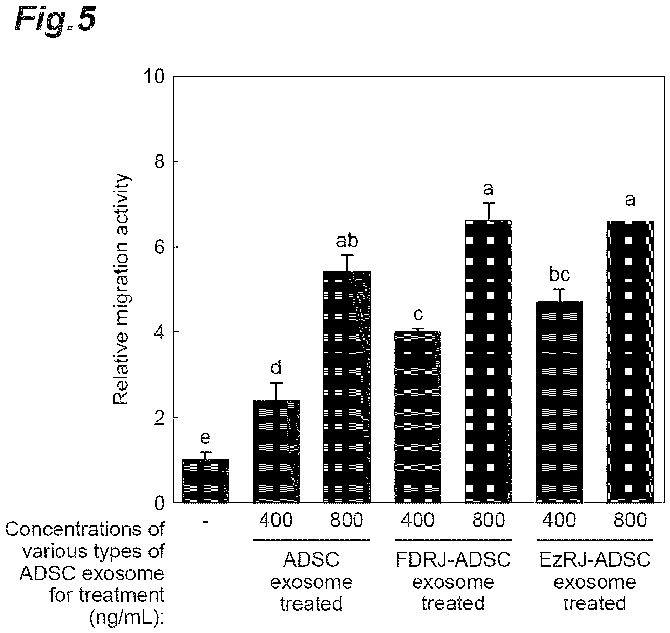

NHDF adjusted at 1 × 10⁵ cells/mL were seeded in a 12-well plate at 1 mL/well, and then pre-cultured in NHDF media (Lonza K.K.) at 37°C and 5%CO₂ for 24 hours. After 24 hours, the media were exchanged with NHDF media, to which various types of ADSC exosome same as those of Experimental Example 2 had been added in such a manner that the concentrations were 400 and 800 ng protein/mL, respectively, and then NHDF were further cultured for 24 hours. After 24 hours of culture, the media were exchanged with NHDF media, and then cell layers were each scratched with a tip of a 1,000-µL chip, thereby forming a linear wound site. After 24 hours of culture in NHDF media, pictures showing the numbers of NHDF that had migrated to the wound sites were taken under a phase-contrast microscope and shown in Fig. 4. Further, the number of cells that had migrated was counted with ImageJ, and then the relative migration activity when the migration activity of untreated NHDF was determined to be 1 was calculated. Fig. 5 shows the results. Note that a, ab, c, be, d, and e in Fig. 5 indicate that there is a significant difference between different symbols. Further, a and ab, and, c and be share the same symbol, indicating that there is no significant difference.

Fig. 4 and Fig. 5 demonstrate that compared with untreated NHDF, the migration activity of NHDF treated with ADSC exosome, and that of NHDF treated with FDRJ-ADSC exosome and that of NHDF treated with EzRJ-ADSC exosome were increased in a concentration- dependent manner. NHDF treated with FDRJ-ADSC exosome or treated with EzRJ-ADSC exosome exhibited relative migration activity increased about two times that of NHDF treated with ADSC exosome.

### (Experimental Example 4 Evaluation of the effects of various types of ADSC exosome on the ability of fibroblasts to produce collagen)

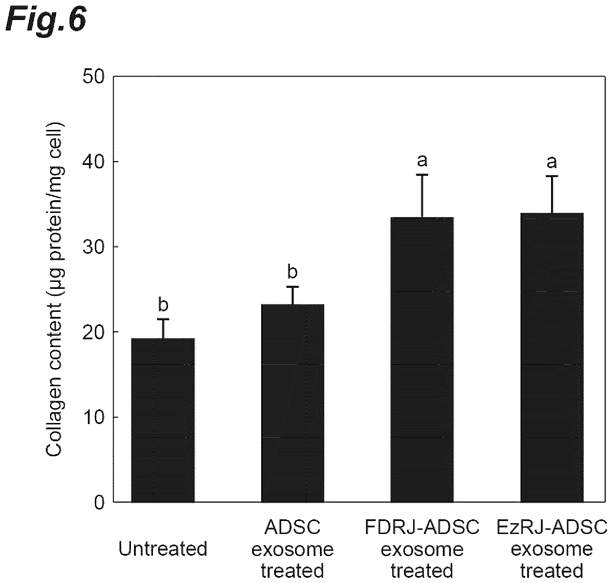

NHDF adjusted at 1 × 10⁵ cells/mL were seeded in a 12-well plate at 1 mL/well, and then pre-cultured in NHDF media (Lonza K.K.) at 37°C and 5%CO₂ for 24 hours. After 24 hours, various types of ADSC exosome same as those in Experimental Example 2 were each added to each medium in such a manner that the concentration was 200 ng protein/mL, followed by 72 hours of culture. After 72 hours of culture, the media were exchanged with NHDF media, and then cells were cultured for 72 hours. After 72 hours of culture, cells were washed with cold PBS, and then the content of the thus generated collagen was quantitatively determined using a Collagen Quantitation Kit. Fig 6 shows the results. Note that a and b in Fig. 6 indicate that there is a significant difference between different symbols. Further, it is also indicated that no significant difference is present between the same symbols.

Fig. 6 demonstrates that no change was observed in intracellular collagen level between cells treated with ADSC exosome and untreated cells. On the other hand, cells treated with FDRJ-ADSC exosome and cells treated with EzRJ-ADSC exosome increased the intracellular collagen content about 1.7 times that of untreated cells. There was no significant difference in intracellular collagen content between cells treated with FDRJ-ADSC exosome and cells treated with EzRJ-ADSC exosome.

### (Experimental Example 5 Evaluation of the effects of various types of ADSC exosome on microRNA (miRNA) profile)

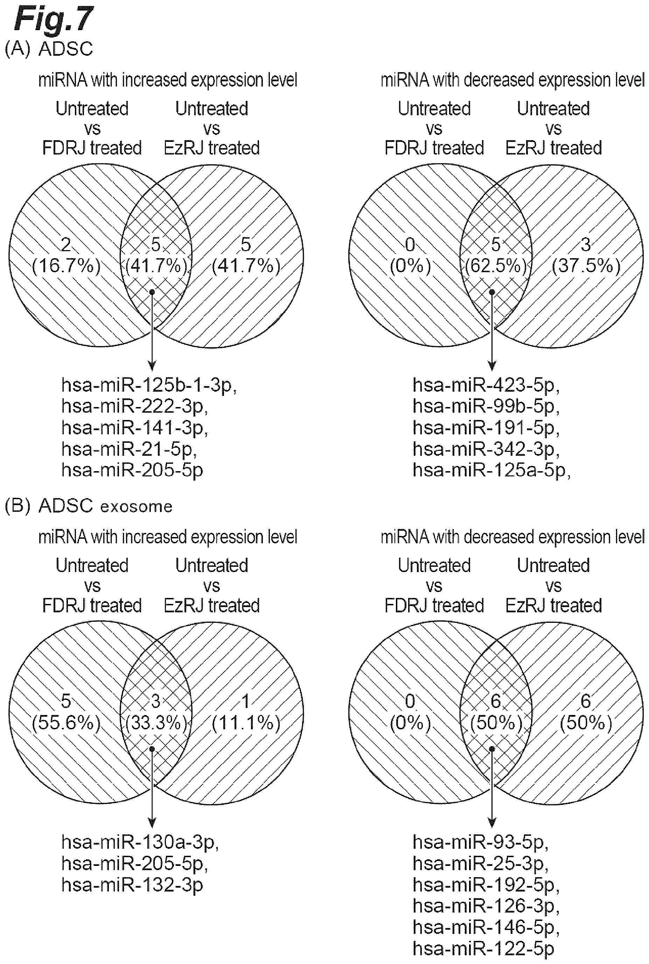

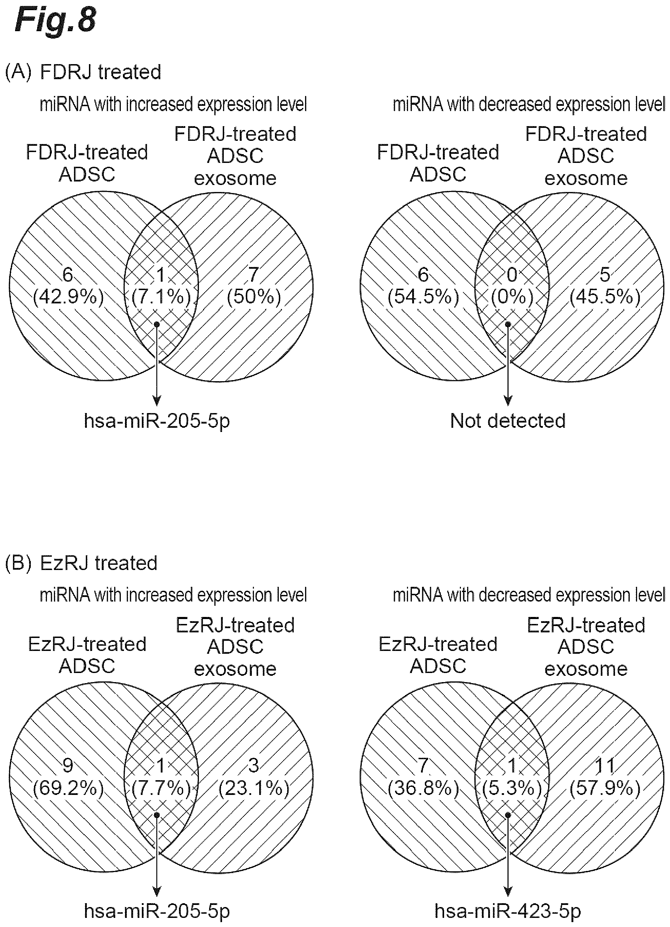

From ADSC and ADSC exosome treated with various types of royal jelly prepared by the method described in Experimental Example 1, total RNA was extracted using a RNeasy Mini Kit (QIAGEN N.V.). RNA samples were confirmed to undergo no degradation, with the use of a Bioanalyzer (Agilent Technologies, Inc.). Using a QIA-seq miRNA Library Kit (QIAGEN N.V.) and QIA-seq miRNA NGS 96 Index IL (QIAGEN N.V.) separately, libraries for miRNA-seq analysis were prepared. Immediately after preparation, the libraries were applied to a next generation sequencer NovaSeq 6000 (Registered Trademark, Illumina) for analyzing the miRNA profile in ADSC and ADSC exosome. Note that data in which the count value of the number of reads from detected miRNA was less than 16 were deleted as noise data, and a total of 94 types of miRNA were subjected to analysis. Table 1 shows the contents of simultaneously differentially expressed miRNAs in ADSC and ADSC exosome. The miRNA contents in Table 1 are represented by TPM (Transcripts per million). Further, Fig. 7 and Fig. 8 are Venn diagrams showing simultaneously differentially expressed miRNA subjected to screening.

Through comparison of untreated ADSC with ADSC treated with various types of royal jelly, and comparison of untreated ADSC exosome with ADSC exosome treated with various types of royal jelly, miRNAs for which a change had been seen due to treatment with various types of royal jelly, were extracted. As a result, compared with untreated ADSC, expression levels of hsa-miR-125b-1-3p, hsa-miR-222-3p, hsa-miR-141-3p, hsa-miR-21-5p, and has-miR-205-5p were commonly increased ADSC treated with various types of royal jelly. Furthermore, expression levels of hsa-miR-422-5p, hsa-miR-99b-5p, hsa-miR-191-5p, hsa-miR-342-5p, and hsa-miR-125-5p were commonly decreased in ADSC treated with various types of royal jelly (Table 1, Fig. 7(A)). On the other hand, compared with untreated ADSC exosome, in ADSC exosome treated with various types of royal jelly, the contents of hsa-miR-130a-3p and hsa-miR-205-5p were commonly increased, and the contents of hsa-miR-95-3p, hsa-miR-25-3p, hsa-miR-192-5p, hsa-miR-126-3p, hsa-miR-146-5p, and hsa-miR-122-5p were commonly decreased (Table 1, Fig. 7(B)). When miRNAs for which a change had been seen in ADSC and ADSC exosome treated with various types of royal jelly, were extracted, the miRNA for which a change had commonly been seen in both ADSC and ADSC exosome, and for which a change had commonly been seen in treatment with various types of royal jelly, was hsa-miR-205-5p alone, for which the expression level was increased (Table 1, Fig. 8).

**[Table 1]**

| miRNA | miRNA content in ADSC (TPM (Log2)) | | | miRNA content in ADSC exosome (TPM (Log2)) | | |
|---|---|---|---|---|---|---|
| | Untreated ADSC | FDRJ -treated ADSC | EzRJ -treated ADSC | Untreated ADSC exosome | FDRJ-ADSC exosome | EzRJ-ADSC exosome |
| hsa-niR-125b-1-3p | 9.4 | 10.6 | 10.4 | - | - | - |
| hsa-miR-222-3p | 10.2 | 11.7 | 11.4 | - | - | - |
| hsa-miR-141-3p | 1.83 | 3.0 | 4.4 | 11.5 | 9.9 | 9.3 |
| hsa-miR-21-5p | 15.6 | 16.7 | 16.7 | - | - | - |
| hsa-miR-423-5p | 9.9 | 8.6 | 8.2 | 12.5 | 10.6 | 10.3 |
| hsa-miR-99b-5p | 12.7 | 11.5 | 11.5 | - | - | - |
| hsa-miR-191-5p | 12.8 | 11.7 | 11.6 | 13.2 | 11.8 | 11.7 |
| hsa-miR-342-3p | 10.0 | 9.0 | 8.9 | 11.9 | 10.1 | 10.4 |
| hsa-miR-125a-5p | 14.6 | 13.5 | 13.5 | - | - | - |
| hsa-miR-130a-3p | - | - | - | 7.8 | 8.9 | 9.4 |
| hsa-miR-205-5p | 3.8 | 5.0 | 4.9 | 9.9 | 11.4 | 11.8 |
| hsa-miR-93-5p | - | - | - | 13.1 | 10.8 | 9.8 |
| hsa-miR-25-3p | - | - | - | 13.3 | 11.0 | 10.4 |
| hsa-miR-192-5p | 6.6 | 6.2 | 8.2 | 13.3 | 10.1 | 10.1 |
| hsa-miR-126-3p | - | - | - | 13.9 | 10.8 | 10.0 |
| hsa-miR-146a-5p | - | - | - | 12.4 | 9.6 | 9.9 |
| hsa-miR-122-5p | 2.7 | 3.1 | 4.9 | 14.0 | 11.3 | 10.6 |
| hsa-miR-132-3p | - | - | - | 7.7 | 9.7 | 9.7 |

## Claims

1. An agent comprising a royal jelly for use in promotion of exosome secretion by mesenchymal stem cells.

2. The agent for use according to claim 1, wherein the mesenchymal stem cells are adipose-derived stem cells.

3. The agent for use according to claim 1 or 2, wherein the royal jelly improves the content of miRNA-205-5p in mesenchymal stem cells and/or the exosome secreted by mesenchymal stem cells.

4. A cosmetic raw material, a cosmetic product, a food composition or a pharmaceutical composition comprising a royal jelly, for use in promotion of exosome secretion by mesenchymal stem cells.

5. A method for producing an exosome derived from mesenchymal stem cells, comprising a step of culturing mesenchymal stem cells in a medium containing royal jelly.

6. The production method according to claim 5, wherein the mesenchymal stem cells are adipose-derived mesenchymal stem cells.

7. The production method according to claim 5 or 6, further comprising a step of collecting a secreted exosome.

8. A composition, comprising an exosome produced by the production method according to any one of claims 5 to 7.

9. The composition according to claim 8, which is a skin care composition.

10. The composition according to claim 8 or 9, having fibroblast activating action.

11. The composition according to claim 10, wherein the fibroblast activating action includes fibroblast growth promoting action, wound healing promoting action, migration promoting action to a wound site, and/or collagen production promoting action.

12. The composition according to any one of claims 8 to 11, which is a cosmetic raw material, a cosmetic product, a food composition or a pharmaceutical composition.

13. The composition according to claim 12, wherein the composition is a topical composition.
